# EUROPEAN PATENT APPLICATION

(11) **EP 3 190 103 A1**
(43) Date of publication of application: **12.07.2017**
(21) Application number: 16000022.0
(22) Date of filing: 08.01.2016
(51) Int. Cl.: C07D 211/34, C07C 233/77, C07D 213/64, A61K 31/44, A61P 35/00

(54) **INHIBITORS OF THE PD-1/PD-L1 PROTEIN/PROTEIN INTERACTION**

(71) Applicant: Rijksuniversiteit Groningen, 9712 CP Groningen (NL)
(72) Inventor: DÖMLING, Alexander, 42653 Solingen (DE)
(74) Representative: Forstmeyer, Dietmar

(57) **Abstract**

The present invention provides novel compounds of formula (I) that are useful as inhibitors of the PD-1/PD-L1 protein/ protein interaction.

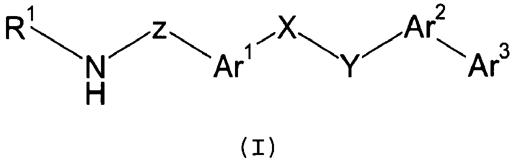

## Description

The present invention provides novel compounds that are useful as inhibitors of the PD-1/PD-L1 protein/protein interaction.

The PD-1/PD-L1 axis is hijacked by viruses and uncontrolled fast growing cells to suppress the immune surveillance. In cancer for example, the malignant cells express PD-L1 which bind to the PD1 receptor expressed on immune T-cells. Binding of PD-1 to PD-1L determines a downregulation of T-cell effector functions in cancer patients inhibiting the antitumor immune response and leading to T-cell exhaustion. In viral diseases a similar mechanism is used by viruses to undermine the effective immune recognitions and answer.

Current medication directed towards the PD-1/PD-L1 axis includes monoclonal antibodies. These have shown impressive clinical results in the treatment of several types of tumours. Therapeutic antibodies however exhibit several disadvantages such as limited tissue and tumour penetration, very long half life time, lacking oral bioavailability, immunogenicity, and difficult and expensive production. The current PD-1/PD-L1 axis directed monoclonal antibodies lead to a tumour response only in a fraction of cases and tumour types. Recently small molecules have been described to bind to PD-L1 in WO 2015/160641 and WO 2015/034820. The compounds described therein, however, display a high lipophilicity (cLogP).

A high cLogP is often associated with extensive metabolism, poor water solubility, fast excretion and toxicity and reduced target selectivity.

Therefore PD-1/PD-L1 axis targeted drugs are needed which overcome the above disadvantages and which further lead to a high tumour response, are fast and efficient to produce and can penetrate tumour tissue and have favourable half-life times to be able to adequately react on drug induced immunological adverse side effects. These objects are solved by the compounds of the present invention.

The present invention provides compounds of formula (I): 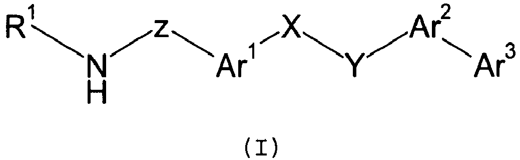 wherein
R¹ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
Ar¹ is an aryl, hereroaryl, aralkyl or heteroaralkyl group, all of which may optionally be substituted;
Ar² is a phenylene group or a heteroarylene group having 5 or 6 ring atoms and 1, 2, 3 or 4 heteroatoms selected from O, S and N, which group Ar² is unsubstituted or substituted by one group R² which is preferably bound to a carbon or a nitrogen atom of Ar² which carbon or nitrogen atom is adjacent to an atom at which group Ar³ is bound to Ar² and to an atom at which group Y is bound to Ar²;
Ar³ is a phenylene group or a heteroarylene group having 5 or 6 ring atoms and 1, 2, 3 or 4 heteroatoms selected from O, S and N which group Ar³ is unsubstituted or substituted by one, two or three group (s) R³;
X is a bond or O, S, CHOMe, NOMe, CFH, CF₂, NH or CH₂;
Y is a bond or O, S, CHOMe, NOMe, CFH, CF₂, NH or CH₂;
z is a bond or a C₁-C₆ alkylene, a C₂-C₆ alkenylene, a C₂-C₆ alkynylene or a heteroalkylene group containing from 2 to 8 atoms selected from C, N, O and S;
R² is methyl, CN or halogen;
the group(s) R³ is/are independently halogen, CN, hydroxy or a C₁-C₆ alkyl, a C₂-C₆ alkenyl, a C₂-C₆ alkynyl or a heteroalkyl group containing from 2 to 8 atoms selected from C, N, O and S or a C₃-C₇ cycloalkyl group or a heterocycloalkyl group containing 3 to 7 ring atoms selected from O, S, N and C; or two groups R³ together are part of a C₃-C₇ cycloalkyl group, a heterocycloalkyl group containing 3 to 7 ring atoms selected from O, S, N and C, a phenyl group or a heteroraryl group having 5 or 6 ring atoms and 1, 2, 3 or 4 heteroatoms selected from O, S and N;
or a pharmaceutically acceptable salt, ester, solvate or hydrate or a pharmaceutically acceptable formulation thereof.

In the following, preferred embodiments of the present invention are disclosed. It is preferred that the preferred embodiments may be combined in any manner:
Preferably, Ar² is not a phenyl group when Ar³ is a phenyl group or a thienyl group and X is O and Y is CH₂.

Further preferably, X is not O, S or NH, when Y is O, S or NH.

Moreover preferably, X is CH₂ and Y is O or X is O and Y is CH₂.

Further preferably, Ar³ is unsubstituted.

Moreover preferably, Ar³ is substituted by a halogen atom, especially F or Cl.

Further preferably, Ar³ is substituted by two groups R³ that together are part of a C₅-C₆ cycloalkyl group or a heterocycloalkyl group containing 5 or 6 ring atoms selected from O, S, N and C.

Especially preferably, Ar³ is substituted by two groups R³ which together form a group of formula -O-CH₂CH₂-O-, -O-CH₂-O-, or -O-CF₂-O-.

Further preferably, Ar² is substituted by a methyl group, i.e. R² is methyl.

Moreover preferably, z is CH₂.

Further prefreably, R¹ is hydrogen, methyl or a group of formula CH₂CH₂OH, CH₂CH₂NH₂ or CH₂CH₂NHCOCH₃.

Further preferably, Ar¹ is selected from the following groups: 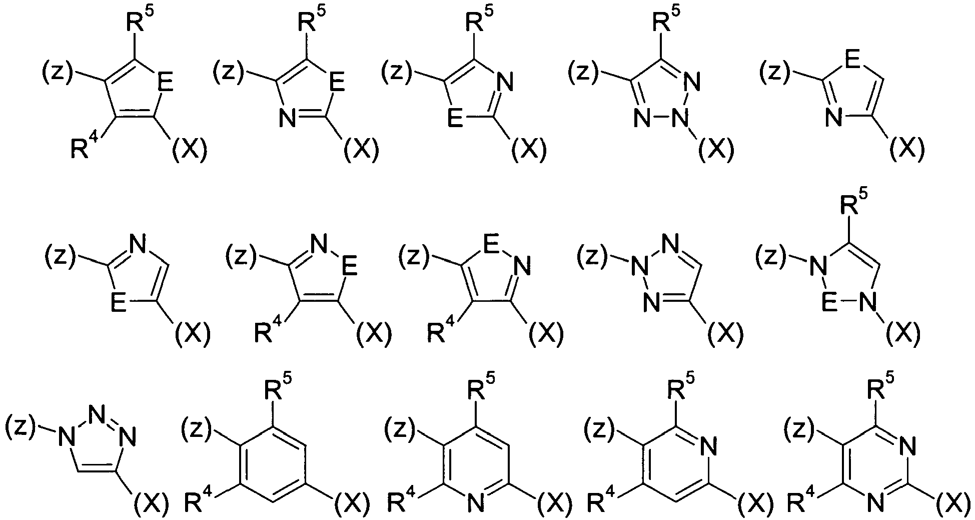 wherein (z) denotes the bond to group z; (X) denotes the bond to group X; E is selected from O, S and NR⁶; and R⁴, R⁵ and R⁶ are independently selected from a hydrogen atom, a halogen atom, NO₂, N₃, OH, SH, NH₂, SO₃H or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

Further preferably, Ar² is selected from the following groups: 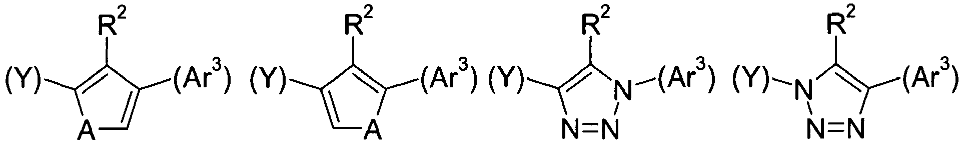 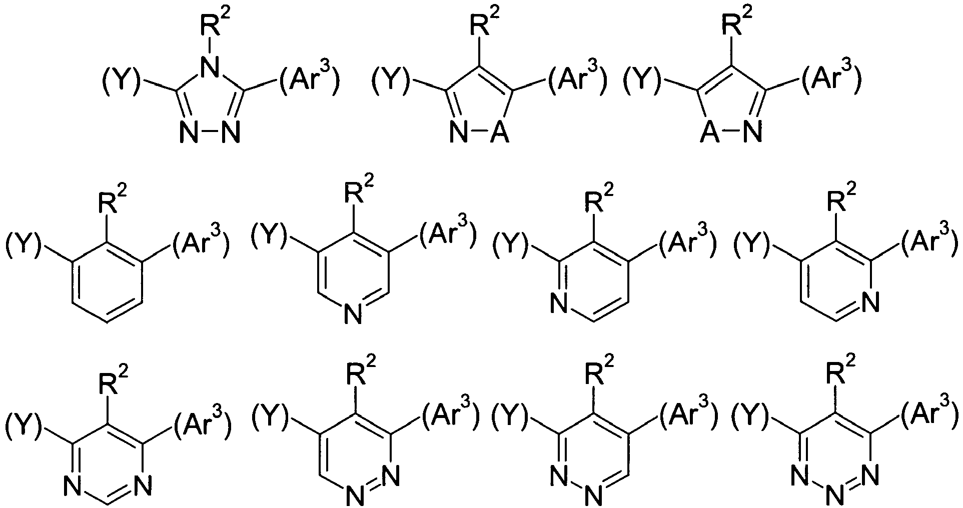 wherein (Y) denotes the bond to group Y; (Ar³) denotes the bond to group Ar³; A is selected from O, S and NH and R² is as defined above.

Further preferably, Ar³ is selected from the following groups: 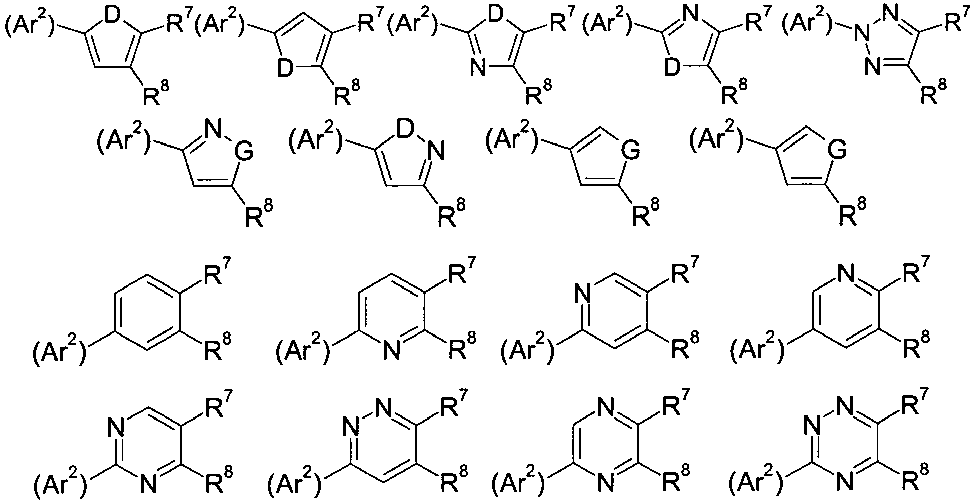 wherein (Ar²) denotes the bond to group Ar²; D is selected from O, S and NR⁹; G is selected from O, S and NR⁷; R⁷, R⁸ and R⁹ are independently hydrogen, halogen, CN, hydroxy or a C₁-C₆ alkyl, a C₂-C₆ alkenyl, a C₂-C₆ alkynyl or a heteroalkyl group containing from 2 to 8 atoms selected from C, N, O and S or a C₃-C₇ cycloalkyl group or a heterocycloalkyl group containing 3 to 7 ring atoms selected from O, S, N and C; or R⁷ and R⁸ together are part of a C₃-C₇ cycloalkyl group, a heterocycloalkyl group containing 3 to 7 ring atoms selected from O, S, N and C, a phenyl group or a heteroraryl group having 5 or 6 ring atoms and 1, 2, 3 or 4 heteroatoms selected from O, S and N.

Especially preferably, R⁷ and R⁸ together form a group of formula -O-CH₂CH₂-O-, -O-CH₂-O-, or -O-CF₂-O-.

The expression alkyl refers to a saturated, straight-chain or branched hydrocarbon group that contains from 1 to 20 carbon atoms, preferably from 1 to 12 carbon atoms, especially from 1 to 6 (e.g. 1, 2, 3 or 4) carbon atoms, for example a methyl, ethyl, propyl, iso-propyl, n-butyl, iso-butyl, sec-butyl, tert-butyl, n-pentyl, iso-pentyl, n-hexyl, 2,2-dimethylbutyl or n-octyl group. Furthermore, the term alkyl refers to groups in which one or more hydrogen atoms have been replaced by a halogen atom (preferably F or Cl) such as, for example, a 2,2,2-trichloroethyl or a trifluoromethyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 (e.g. 2, 3 or 4) carbon atoms, for example an ethenyl (vinyl), propenyl (allyl), iso-propenyl, butenyl, ethinyl, propinyl, butinyl, acetylenyl, propargyl, isoprenyl or hex-2-enyl group. Preferably, alkenyl groups have one or two (especially preferably one) double bond(s), and alkynyl groups have one or two (especially preferably one) triple bond(s). Furthermore, the terms alkenyl and alkynyl refer to groups in which one or more hydrogen atoms have been replaced by a halogen atom (preferably F or Cl).

The expression heteroalkyl refers to an alkyl, alkenyl or alkynyl group in which one or more (preferably 1, 2 or 3) carbon atoms have been replaced by an oxygen, nitrogen, phosphorus, boron, selenium, silicon or sulfur atom (preferably by an oxygen, sulfur or nitrogen atom). The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy.

Preferably, a heteroalkyl group contains from 1 to 12 carbon atoms and from 1 to 4 hetero atoms selected from oxygen, nitrogen and sulphur (especially oxygen and nitrogen). Especially preferably, a heteroalkyl group contains from 1 to 6 (e.g. 1, 2, 3 or 4) carbon atoms and 1, 2 or 3 (especially 1 or 2) hetero atoms selected from oxygen, nitrogen and sulphur (especially oxygen and nitrogen). The term C₁-C₆ heteroalkyl refers to a heteroalkyl group containing from 1 to 6 carbon atoms and 1, 2 or 3 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N). Furthermore, the term heteroalkyl refers to groups in which one or more hydrogen atoms have been replaced by a halogen atom (preferably F or Cl).

Examples of heteroalkyl groups are groups of formulae: R^{a}-O-Y^{a}-, R^{a}-S-Y^{a}-, R^{a}-N(R^{b}) -Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N (R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a direct bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, n-propyloxy, isopropyloxy, butoxy, tert-butyloxy, methoxymethyl, ethoxymethyl, -CH₂CH₂OH, -CH₂OH, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile, isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group.

The expression heterocycloalkyl refers to a cycloalkyl group as defined above in which one or more (preferably 1, 2, 3 or 4) ring carbon atoms have been replaced by an oxygen, nitrogen, silicon, selenium, phosphorus or sulfur atom (preferably by an oxygen, sulfur or nitrogen atom). A heterocycloalkyl group has preferably 1 or 2 ring(s) containing from 3 to 10 (especially 3, 4, 5, 6 or 7) ring atoms (preferably secected from C, O, N and S). The expression heterocycloalkyl refers furthermore to groups in which one or more hydrogen atoms have been replaced by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl, urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings having from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2, 3, 4 or 5) carbon atoms have been replaced by an oxygen, nitrogen, silicon, selenium, phosphorus or sulfur atom (preferably by an oxygen, sulfur or nitrogen atom). A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings containing from 6 to 14 ring carbon atoms, preferably from 6 to 10 (especially 6) ring carbon atoms. The expression aryl refers furthermore to groups in which one or more hydrogen atoms have been replaced by fluorine, chlorine, bromine or iodine atoms or by OH, SH, NH₂, N₃ or NO₂ groups. Examples are the phenyl, naphthyl, biphenyl, 2-fluorophenyl, anilinyl, 3-nitrophenyl or 4-hydroxyphenyl group.

The expression heteroaryl refers to an aromatic group that contains one or more rings containing from 5 to 14 ring atoms, preferably from 5 to 10 (especially 5 or 6 or 9 or 10) ring atoms, and contains one or more (preferably 1, 2, 3, 4 or 5) oxygen, nitrogen, phosphorus or sulfur ring atoms (preferably O, S or N). The expression heteroaryl refers furthermore to groups in which one or more hydrogen atoms have been replaced by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl,thiadiazolyl, indolyl, indazolyl, tetrazolyl, pyrazinyl, pyrimidinyl, pyridazinyl, oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetraline, dihydronaphthalene, indanone, phenylcyclopentyl, cumene, cyclohexylphenyl, fluorene and indane. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulfur atom (preferably oxygen, sulfur or nitrogen), that is to say to groups containing both aryl or heteroaryl, respectively, and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, wherein 1, 2, 3, 4, 5 or 6 of these carbon atoms have been replaced by oxygen, sulfur or nitrogen atoms. Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, 2-or 3-ethylindolyl, 4-methylpyridino, 2-, 3- or 4-methoxyphenyl, 4-ethoxyphenyl, 2-, 3- or 4-carboxyphenylalkyl group.

The term halogen or halogen atom refers to F, Cl, Br or I.

The expression "optionally substituted" especially refers to groups in which one, two, three or more hydrogen atoms may have been replaced by fluorine, chlorine, bromine or iodine atoms or by OH, =O, SH, =S, NH₂, =NH, N₃ or NO₂ groups. This expression refers furthermore to groups that may be substituted by one, two, three or more preferably unsubstituted C₁-C₁₀ alkyl, C₂-C₁₀ alkenyl, C₂-C₁₀ alkynyl, C₁-C₁₀ heteroalkyl, C₃-C₁₈ cycloalkyl, C₂-C₁₇ heterocycloalkyl, C₄-C₂₀ alkylcycloalkyl, C₂-C₁₉ heteroalkylcycloalkyl, C₆-C₁₈ aryl, C₁-C₁₇ heteroaryl, C₇-C₂₀ aralkyl or C₂-C₁₉ heteroaralkyl groups. This expression refers furthermore especially to groups that may be substituted by one, two, three or more preferably unsubstituted C₁-C₆ alkyl, C₂-C₆ alkenyl, C₂-C₆ alkynyl, C₁-C₆ heteroalkyl, C₃-C₁₀ cycloalkyl, C₂-C₉ heterocycloalkyl, C₇-C₁₂ alkylcycloalkyl, C₂-C₁₁ heteroalkylcycloalkyl, C₆-C₁₀ aryl, C₁-C₉ heteroaryl, C₇-C₁₂ aralkyl or C₂-C₁₁ heteroaralkyl groups.

If a substituent contains a ring, this ring may be bonded to the respective substituted group via a single or double bond (especially a single bond) or, if the substituted group also contains a ring, the ring of the substituent may also be annulated to the ring of the substituted group.

Preferred substituents are F, Cl, Br, OH, =O, NH₂, C₁₋₄ alkyl (e.g. methyl, ethyl, t-butyl), NMe₂, CONH₂, CH₂NMe₂, NHSO₂Me, C(CH₃)₂CN, COMe, OMe, SMe, COOMe, COOEt, CH₂COOH, OCH₂COOH, COOH, SOMe, SO₂Me, cyclopropyl, SO₂NH₂, SO₂NHMe, SO₂CH₂CH₂OH, SF₅, SO₂NMe₂, OCF₃, SO₂CF₃, COMe, CN or CF₃.

Especially preferred substituents are F, Cl, Br, OH, NH₂, Me, Ethyl, NMe₂, CONH₂, OMe, CN or CF₃.

According to a preferred embodiment, all alkyl, alkenyl, alkynyl, heteroalkyl, aryl, heteroaryl, cycloalkyl, heterocycloalkyl, alkylcycloalkyl, heteroalkylcycloalkyl, aralkyl and heteroaralkyl groups described herein may optionally be substituted.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated.

The present invention further provides pharmaceutical compositions comprising one or more compounds of formula (I) as defined herein or a pharmaceutically acceptable ester, prodrug, hydrate, solvate or salt thereof, optionally in combination with a pharmaceutically acceptable carrier.

It is a further object of the present invention to provide a compound of formula (I) as defined herein or a pharmaceutical composition as defined herein for the preparation of a medicament for the treatment of one or more diseases mentioned herein.

Preferably the compounds of the present invention may be used for the treatment and/or prevention of cancer and infectious diseases.

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage may be adjusted to the individual requirements in each particular case including the specific compound being administered, the route of administration, the condition being treated, as well as the patient being treated.

Examples of pharmacologically acceptable salts of sufficiently basic compounds of formula (I) are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound of formula (I) may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of formula (I). Compounds of formula (I) may be solvated, especially hydrated. The hydratization/hydration may occur during the process of production or as a consequence of the hygroscopic nature of the initially water free compounds of formula (I). The solvates and/or hydrates may e.g. be present in solid or liquid form.

It should be appreciated that certain compounds of formula (I) may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). All these tautomeric forms, geometrical or optical isomers (as well as racemates and diastereomers) and polymorphous forms are included in the invention. Since the compounds of formula (I) may contain asymmetric C-atoms, they may be present either as achiral compounds, mixtures of diastereomers, mixtures of enantiomers or as optically pure compounds. The present invention comprises both all pure enantiomers and all pure diastereomers, and also the mixtures thereof in any mixing ratio.

The therapeutic use of compounds according to formula (I), their pharmacologically acceptable salts, solvates and hydrates, respectively, as well as formulations and pharmaceutical compositions also lie within the scope of the present invention.

The pharmaceutical compositions according to the present invention comprise at least one compound of formula (I) as an active ingredient and, optionally, carrier substances and/or adjuvants.

The present invention also relates to pro-drugs which are composed of a compound of formula (I) and at least one pharmacologically acceptable protective group which will be cleaved off under physiological conditions, such as an alkoxy-, arylalkyloxy-, acyl-, acyloxymethyl group (e.g. pivaloyloxymethyl), an 2-alkyl-, 2-aryl- or 2-arylalkyloxycarbonyl-2-alkylidene ethyl group or an acyloxy group as defined herein, e.g. ethoxy, benzyloxy, acetyl or acetyloxy or, especially for a compound of formula (I), carrying a hydroxy group (-OH): a sulfate, a phosphate (-OPO₃ or - OCH₂OPO₃) or an ester of an amino acid.

Preferably, the present invention also relates to a prodrug, a biohydrolyzable ester, a biohydrolyzable amide, a polymorph, tautomer, stereoisomer, metabolite, N-oxide, biohydrolyzable carbamate, biohydrolyzable ether, physiologically functional derivative, atropisomer, or in vivo-hydrolysable precursor, diastereomer or mixture of diastereomers, chemically protected form, affinity reagent, complex, chelate and a stereoisomer of the compounds of formula (I).

As used herein, the term pharmaceutically acceptable ester especially refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates.

As mentioned above, therapeutically useful agents that contain compounds of formula (I), their solvates, salts or formulations are also comprised in the scope of the present invention. In general, compounds of formula (I) will be administered by using the known and acceptable modes known in the art, either alone or in combination with any other therapeutic agent.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 10 mg to about 10,000 mg, preferably from about 20 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

The present invention moreover provides a method of inhibiting growth, proliferation, or metastasis of cancer cells in a subject in need thereof, said method comprising administering to the subject a therapeutically effective amount of a compound of formula (I), or a pharmaceutically acceptable salt. In one embodiment the cancer is selected from melanoma, renal cell carcinoma, squamous non-small cell lung cancer (NSCLC), non-squamous NSCLC, colorectal cancer, castration-resistant prostate cancer, ovarian cancer, gastric cancer, hepatocellular carcinoma, pancreatic carcinoma, squamous cell carcinoma of the head and neck, carcinomas of the esophagus, gastrointestinal tract and breast, cancer of the genital organs, penis and vagina, and a hematological malignancy.

Further the present invention provides a method of treating an infectious disease in a subject in need thereof, the method comprising administering to the subject a therapeutically effective amount of a compound of formula (I), or a pharmaceutically acceptable salt thereof. In one embodiment the infectious disease is caused by a virus. In a further embodiment the virus is selected from HIV, Hepatitis A, Hepatitis B, Hepatitis C, Hepatitis D, herpes viruses, papillomaviruses and influenza.

In one embodiment, a method is provided for treating cancer comprising administering to a patient in need thereof, a therapeutically effective amount of a compound of formula (I) or a salt thereof. Examples of cancers include those whose growth may be inhibited using compounds of the disclosure include cancers typically responsive to immunotherapy. Nonlimiting examples of preferred cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, the disclosure includes refractory or recurrent malignancies whose growth may be inhibited using the compounds of the present invention.

Examples of other cancers that may be treated using the methods of the present invention include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or urethra, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. The present invention is also useful for treatment of metastatic cancers, especially metastatic cancers that express PD-L1.

Some examples of pathogenic viruses causing infections treatable by methods of the present invention include HIV, hepatitis (A, B, C, or D), herpes viruses (e.g., VZV, HSV-1 , HAV-6, HHv-7, HHV-8, HSV-2, CMV, and Epstein Barr virus), adenovirus, influenza virus, fiaviviruses, echovirus, rhinovirus, coxsackie virus, cornovirus, respiratory syncytial viras, mumps viras, rotaviras, measles viras, rabella viras, parvovirus, vaccinia virus, HTLV viras, dengue viras, papillomavirus, molluscum viras, poliovirus, rabies viras, JC viras and arboviral encephalitis viras.

Some examples of pathogenic bacteria causing infections treatable by methods of the present invention include chlamydia, rickettsial bacteria, mycobacteria, staphylococci, streptococci, pneumonococci, meningococci and conococci, lebsiella, proteus, serratia, pseudomonas, legionella, diphtheria, salmonella, bacilli, cholera, tetanus, botulism, anthrax, plague, leptospirosis, and Lymes disease bacteria.

Some examples of pathogenic fungi causing infections treatable by methods of the present invention include Candida (albicans, krasei, glabrata, tropicalis, etc.), Cryptococcus neoformans, Aspergillus (fumigatus, niger, etc.), Genus Mucorales (mucor, absidia, rhizophus), Sporothrix schenkii, Blastomyces dermatitidis, Paracoccidioides brasiliensis, Coccidioides immitis and Histoplasma capsulatum.

Some examples of pathogenic parasites causing infections treatable by methods of the present invention include Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma bracei, Trypanosoma crazi, Leishmania donovani, Toxoplasma gondi, and Nippostrongylus brasiliensis.

### Examples

### Example 1: 1-(benzyloxy)-3-bromo-2-methylbenzene

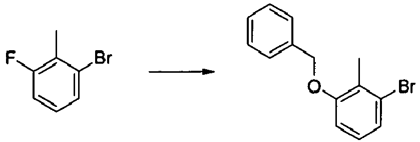

Sodium benzyloxide was prepared by adding benzyl alcohol (20 mmol) to a suspension of NaH (20 mmol) in N-methylpyrrolidone. The freshly prepared solution (10.8 mmol) was added to 1-bromo-3-fluoro-2-methylbenzene (5.41 mmol) in N - methylpyrrolidone. The reaction was heated at 100°C and was monitored by TLC until complete consumption of the starting material. Water and ethyl acetate were added, the aqueous layer was separated and the organic layer was washed with water, dried over MgSO4, filtered and concentrated. The residue was chromatographed with hexane/EtOAc mixture. Yield: 92%. 1H NMR (500 MHz, CDC13) δ 7.49-7.39 (m, 4H), 7.37 (d, J = 7.0 Hz, 1H), 7.20 (d, J = 8.0 Hz, 1H), 7.02 (t, J = 8.1 Hz, 1H), 6.86 (d, J = 8.2 Hz, 1H), 5.09 (s, 2H), 2.41 (d, J = 3.3 Hz, 3H). 13C NMR (126 MHz, CDC13) δ 157.5, 137.0, 128.6, 128.0, 127.3, 127.3, 127.2, 126.0, 125.0, 110.7, 70.5, 16.0. MS (EI) m/z 277 (M+).
Ref: J. R. Rodriguez, J. Agejas, A. Bueno, Tet. Lett. 2006, 47, 5661-5663

### Example 2: 3-(benzyloxy)-2-methyl-1,1'-biphenyl

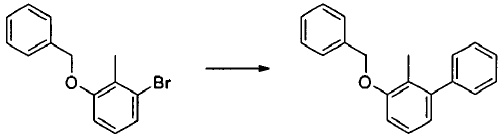

A solution of 1-(benzyloxy)-3-bromo-2-methylbenzene (3.60 mmol), phenylboronic acid (4.32 mmol), sodium carbonate (9 mmol) and tetrakis(triphenylphosphine) palladium (0.36 mmol) in DME (19.5 ml) and water (6.5 ml) were stirred at reflux overnight. The reaction mixture was cooled to room temperature, poured into 100 mL of 1 N NH4C1, and extracted with ethyl acetate (2 x 100 mL). The combined organic layers were washed with water, washed with brine, dried over sodium sulfate, concentrated, and purified on silica to yield 65% as a colorless oil. 1H NMR (500 MHz, DMSO-d6) δ 7.49 (d, J = 7.6 Hz, 2H), 7.46-7.28 (m, 8H), 7.21 (t, J = 7.9 Hz, 1H), 7.05 (d, J = 8.2 Hz, 1H), 6.82 (d, J = 7.6 Hz, 1H), 5.16 (s, 2H), 2.10 (s, 3H). 13C NMR (126 MHz, DMSO-d6) δ 157.0, 143.1, 141.7, 137.9, 129.5, 129.2, 128.9, 128.6, 128.2, 127.9, 127.4, 126.7, 123.9, 122.5, 111.2, 69.9. MS (EI) m/z 275(M+).
Ref: H. A. Harris et al. J. Med. Chem., 2005, 48, 3953-3979

### Example 3: 2-methyl-[1,1'-biphenyl]-3-ol

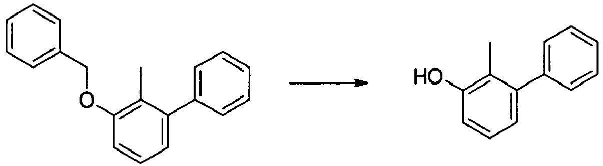

3-(benzyloxy)-2-methyl-1,1'-biphenyl was hydrogenated under atmospheric hydrogen with 10% Pd/C on carbon (containing 50% water, 50 mg) as catalyst in MeOH (5 ml) and THF (2 ml) at room temperature for 72 hours. Filtration of precipitated, wash with ethyl acetate, then concentration gave the colorless oil 299 mg (95%). 1H NMR (500 MHz, CDC13) δ 7.40 (m, 2H), 7.36-7.27 (m, 3H), 7.10 (td, J = 7.8, 2.0 Hz, 1H), 6.89-6.82 (m, 1H), 6.79 (m, 1H). 13C NMR (126 MHz, CDC13) δ 154.1, 143.8, 141.7, 129.3, 128.1, 126.9, 126.3, 122.5, 113.9, 113.9, 13.10. MS (EI) m/z 185 (M+), 207 [M+Na]+.
Ref: S. Mikami et al. J. Med. Chem., 2012, 55, 3756-3776

### Example 4: 2-oxo-4-phenyl-1,2-dihydropyridine-3-carbonitrile

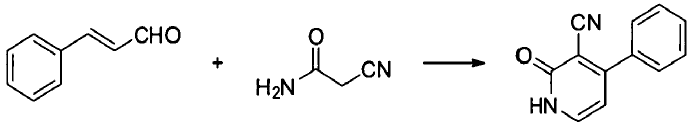

Addition of 4 mol equiv. of t-BuOK, in one portion, to a 0.5 M DMSO solution of cinnamaldehyde (1 equiv.) and 2-cyanoacetamide (1.1. equiv.), at room temperature, and under an oxygen atmosphere (02 balloon), induced an exothermic reaction. After stirring for 30 min without external cooling, the reaction mixture was diluted with 4 volumes of water followed by 5 volumes of 4N aqueous HCI, added slowly and with good stirring. The precipitate was filtered to give the desired pyridine, which was further washed with water, and dried in air, giving a crude product of 65 % yield. Pure product was recrystallized from MeOH. 1H NMR (500 MHz, DMSO-d6) δ 12.62 (br, 1H), 7.82 (d, J = 6.7 Hz, 1H), 7.63 (m, 2H), 7.56 (m, 3H), 6.44 (d, J = 6.7 Hz, 1H). 13C NMR (126 MHz, CDC13) δ 160.8, 160.6, 140.4, 135.9, 130.5, 128.9, 128.0, 116.3, 106.7, 100.9. MS (EI) m/z 197 (M+).
Ref: R. Jain, F. Roschangar, M. A. Ciufolini, Tet. Lett. 1995, 36, 330-3310

### Example 5: Ethyl 2-((3-cyano-4-phenylpyridin-2-yl)oxy)acetate

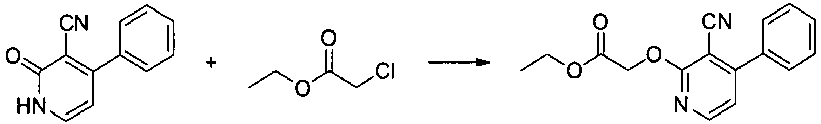

Ethyl 2-chloroacetate (1mmol) was added dropwise to a well-stirred mixture of powdered K2CO3 (1.5 mmol) and 2-oxo-4-phenyl-1,2-dihydropyridine-3-carbonitrile (1mmol) in acetone (25mL). The reaction mixture was heated under reflux for 10-12h. After completion of the reaction water and ethyl acetate were added, the aqueous layer was separated and the organic layer was washed with brine, dried over MgSO4, filtered and concentrated. The residue was chromatographed with petroleum ether/ ethyl acetate, to give a brown solid in 75% yield. 1H NMR (500 MHz, CDC13) δ 7.62 (m, 2H), 7.57-7.45 (m, 4H), 6.40 (d, J = 7.1 Hz, 1H), 4.72 (s, 2H), 4.26 (q, J = 7.1 Hz, 2H), 1.30 (t, J = 7.2 Hz, 3H). 13C NMR (126 MHz, CDC13) δ 167.0, 160.5, 160.3, 141.6, 135.3, 130.90, 129.0, 128.1, 115.4, 107.5, 102.6, 62.4, 50.6, 14.1. MS (EI) m/z 283 (M+), 305 [M+Na] +.
Ref: a) N. S. El-Gohary, M I. Shaaban Arch. Pharm., 2015, 348, 666-680 b)H. C. Shah, V. H. Shah, N. D. Desai, Synth. Comm. 2009, 39, 3126-3140

### Example 6: 2-chloro-3'-methoxy-1,1'-biphenyl

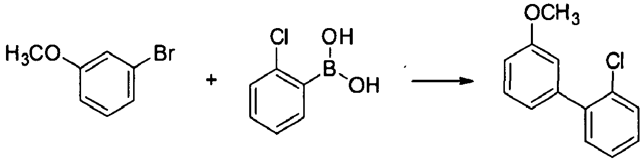

A solution of 1-bromo-3-methoxybenzene (5.34 mmol), (2-chlorophenyl)boronic acid (6.4 mmol), sodium carbonate (13.35 mmol) and tetrakis (triphenylphosphine)palladium (0.53 mmol) in DME (19.5ml) and water (6.5 ml) were stirred under reflux overnight. The reaction mixture was cooled to room temperature, poured into 100 mL of 1 N NH4C1, and extracted with ethyl acetate (2 x 100 mL). The combined organic layers were washed with water, washed with brine, dried over sodium sulfate, concentrated, and purified on silica to yield 81% of a colorless oil. 1H NMR (500 MHz, CDC13) δ 7.52 (d, J = 7.5 Hz, 1H), 7.39 (m, 2H), 7.33 (m, 2H), 7.11-7.03 (m, 2H), 6.99 (d, J = 8.2 Hz, 1H), 3.88 (s, 3H). 13C NMR (126 MHz, CDC13) δ 159.3, 140.8, 140.4, 132.5, 131.3, 130.0, 129.1, 128.6, 126.8, 121.9, 115.2, 113.3, 55.32. MS (EI) m/z 219 (M+).
Ref: H. A. Harris et al. J. Med. Chem., 2005, 48, 3953-3979

### Example 7: 2-amino-4-phenylthiophene-3-carbonitrile

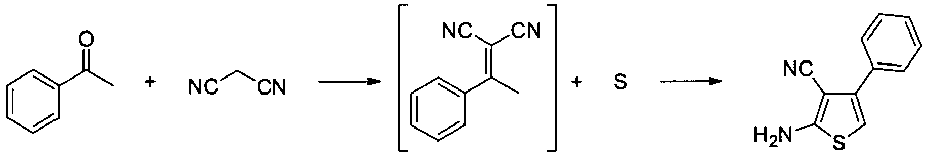

First Step: Malononitrile (32 mmol) and the acetophenone (28 mmol) were dissolved in 20 mL of toluene containing ammonium acetate (500 mg, 6.5 mmol) and glacial acetic acid (2 mL) in a 50 mL flask. By refluxing vigorously, the water formed in the reaction was removed by a Dean and Stark trap placed under the reflux condenser. Evaporation of the toluene left a residue that was recrystallized from alcohol or distilled under vacuum to give pure product. 2-(1-Phenylethylidene) malononitrile Yield: 61%. 1H NMR (CDC13, 300 MHz), δ (ppm) 7.56-7.50 (m, 5H, ArH), 2.63 (s, 3H); 13C NMR (CDC13, 75 MHz), δ (ppm) 175.4, 135.7, 132.1, 128.9, 127.2, 112.7, 112.6, 84.5, 24.1.

Second step : A 20mL vial is charged with 2-(1-Phenylethylidene) malononitrile (5 mmol), sulfur (5 mmol), and triethylamine (5 mmol) in ethanol (5 mL, 1.0 M solution). The reaction is heated 60 ° C for 12 h. Then, the reaction was cooled down to room temperature. Evaporation of ethanol left a residue that was purified by column chromatography Yield: 78%. 1H NMR (500 MHz, CDC13) δ 7.60- 7.50 (m, 2H), 7.39 (m, 2H), 7.36-7.29 (m, 1H), 6.29 (s, 1H), 5.06 (br, 2H). 13C NMR (126 MHz, CDC13) δ 164.0, 139.8, 134.1, 128.7, 128.1, 127.1, 116.1, 105.8, 87.9. MS (EI) m/z 201 (M+).
Ref: a) K. Wang, K. Nguyen, Y. Huang, A. Dömling, J. Comb. Chem., 2009, 11, 920-927 b) K. Wang, D. Kim A. Dömling, J. Comb. Chem., 2010, 12, 111-118

### Example 8: 2-((3-cyano-4-phenylthiophen-2-yl)amino)acetic acid

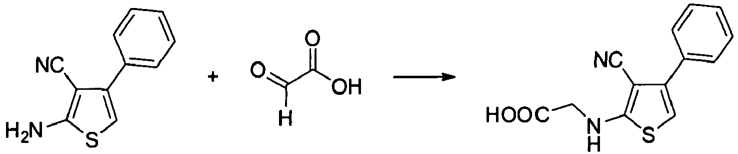

A solution of 2-amino-4-phenylthiophene-3-carbonitrile (10 mmol), glyoxylic acid hydrate (15 mmol) and sodium methoxide (15 mmol) in 100ml. Of absolute methanol was stirred at 65oC for 30 min. After cooling to room temperature, the reaction was further cooled in an ice bath and the imine reduced with portionwise additions of sodium borohydride (15 mmol). Upon complete addition, the ice bath was removed and the mixture stirred for an additional 20 minutes. Methanol was removed in vacuo. The residue was dissolved in a saturated solution of sodium bicarbonate, filtered, and acidified with hydrochloric acid. This method resulted in a yield of Yield: 60%. 1H NMR (500 MHz, CDC13) δ 7.53 (m, 2H), 7.37 - 7.28 (m, 2H), 7.29 - 7.20 (m, 1H), 6.65 (s, 1H), 4.11 (d, J = 6.2 Hz, 2H). 13C NMR (125 MHz, CDC13) δ 171.5, 162.0, 133.5, 132.3, 129.0, 128.8, 128.3, 115.2, 105.1, 91.3, 51.9. MS (EI) m/z 257 (M-).
Ref: R.A. Crochet, J.T. Boatright, C.D. Blanton, J.Het.Chem., 1974, 11, 143-150

### Example 9: 2-amino-5-methyl-4-phenylthiophene-3-carbonitrile

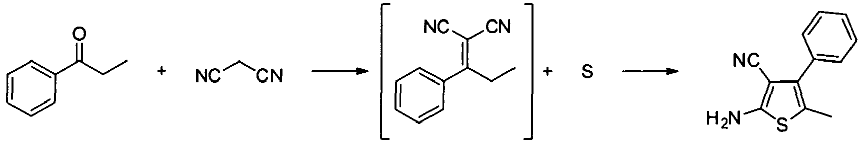

First Step: Malononitrile (32 mmol) and the propiophenone (28 mmol) were dissolved in 20 mL of toluene containing ammonium acetate (500 mg, 6.5 mmol) and glacial acetic acid (2 mL) in a 50 mL flask. By refluxing vigorously, the water formed in the reaction was removed by a Dean and Stark trap placed under the reflux condenser. Evaporation of the toluene left a residue that was recrystallized from alcohol or distilled under vacuum to give pure product. 2-(1-Phenylpropylidene)malononitrile Yield: 65%; solid 1H NMR (CDC13, 300 MHz), δ (ppm) 7.53-7.40 (m, 5H, ArH), 2.98 (q, J = 7.5 Hz, 2H, CH2), 1.11 (t, J = 7.5 Hz, 3H, CH3); 13C NMR (CDC13, 75 MHz), δ (ppm) 181.5, 134.5, 131.9, 129.1, 127.4, 112.6, 112.3, 31.0, 12.7.

Second step: A 20mL vial is charged with 2-(1-Phenylpropylidene)malononitrile (5 mmol), sulfur (5 mmol), and triethylamine (5 mmol) in ethanol (5 mL, 1.0 M solution). The reaction is heated 60 ° C for 12 h. Then, the reaction was cooled down to room temperature. Evaporation of ethanol left a residue that was purified by column chromatography Yield: 81%. 1H NMR (500 MHz, CDC13) δ 7.46-7.40 (m, 2H), 7.38-7.33 (m, 3H), 4.70 (br, 2H), 2.24 (s, 3H). 13C NMR (126 MHz, CDC13) δ 159.9, 135.1, 133.7, 129.1, 128.5, 127.8, 119.2, 115.9, 89.8, 13.4. MS (EI) m/z 215 (M+).
Ref: a) K. Wang, K. Nguyen, Y. Huang, A. Dömling, J. Comb. Chem., 2009, 11, 920-927 b) K. Wang, D. Kim A. Dömling, J. Comb. Chem., 2010, 12, 111-118

### Example 10: 2-amino-5-(chloromethyl)-4-phenylthiophene-3-carbonitrile

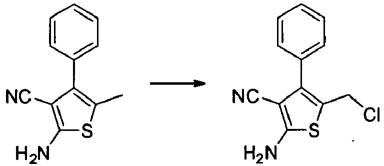

2-amino-5-methyl-4-phenylthiophene-3-carbonitrile (30 mmol) was dissolved in CC14 (150 mL) and the solution was heated to reflux. Benzoyl peroxide (60 mg, 0.25 mmol) was added to the refluxing mixture. After 5 min another batch of benzoyl peroxide (60 mg, 0.25 mmol) and N-bromosuccinimide (5.34 g, 30 mmol) were added. The solution was refluxed for 1 h. After cooling to rt, the reaction mixture was diluted by hexanes. The precipitate was removed via filtration. 2-amino-5-(chloromethyl)-4-phenylthiophene-3-carbonitrile was obtained upon solvent removal.
Ref: A. H. Younes, L. Zhang, R. J. Clark, M. W. Davidson, L. Zhu, Org. Biomol. Chem., 2010, 8, 5431-5441

### Example 11: Methyl 2-((2-methyl-[1,1'-biphenyl]-3-yl)oxy)acetate

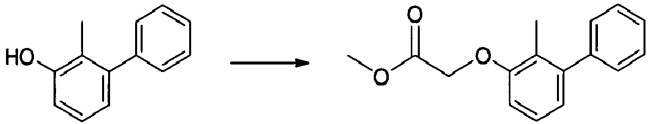

### Example 12: 2-((2-methyl-[1,1'-biphenyl]-3-yl)oxy)acetic acid

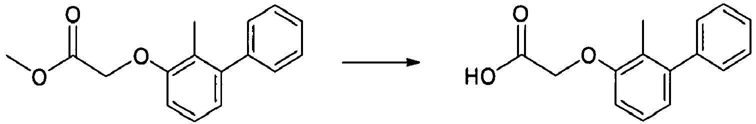

### Example 13: 2-((3-cyano-4-phenylpyridin-2-yl)oxy)acetic acid

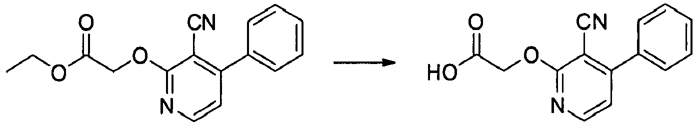

### Example 14: 4-fluoro-3-(2-oxoethoxy)benzonitrile

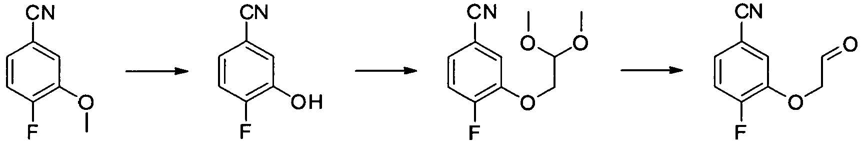

LiCl (3.36g, 79.6 mmol) was added to a solution of 4-fluoro-3-methoxybenzonitrile (3.0 g, 19.9 mmol) in DMF (25 mL). The reaction mixture was refluxed for 16 h. After cooling to rt, the reaction mixture was poored into water, acidified with 6 N HCl and extracted with with EtOAc (3 x 50 mL). The organic layer was sequentially wash with brine (2 x 75 mL), dried over anhydrous Na2SO4, and concentrated in vacuo. The residue was purified by column chromatography (SiO2, hexane/EtOAc 60:40) to give 4-fluoro-3-hydroxybenzonitrile (2.6 g, 96%).
Ref: W. L. Jorgensen, et al J. Med. Chem., 2011, 54, 8582-8591

### Synthesis of reference compounds from US 2015 0291549:

### Example 15: Preparation of (3-bromo-2-methylphenyl)-methanol (2)

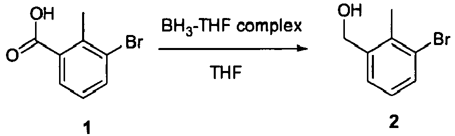

Compound 1 (5.0 g, 23.2 mmol) was dissolved in anhydrous THF (25 mL) under argon and the reaction vessel was cooled to 0 °C in an ice bath. To this cooled solution BH3-THF complex (1M in THF, 35 mL) was added dropwise over a 3 h period. The reaction mixture was warmed to room temperature and stirred for an additional 12 h. The mixture was then poured into 1M hydrochloric acid (126 mL) and then extracted with Et2O (3 x 50 mL). The organic extracts were combined, dried over anhydrous MgSO4, filtered, and concentrated to afford the intermediate (4.6 g; 99 %) as a colorless solid. The crude product was subjected to the subsequent reaction without further purification.

### Example 16: Preparation of (2-Methyl-3-biphenylyl)methanol (4)

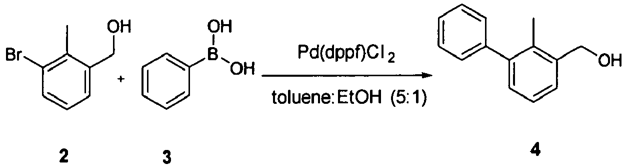

A mixture of compound 2 (4.6 g, 22.8 mmol), phenylboronic acid 3 (5.65 g, 46.3 mmol) and [1,1'-bis(diphenylphosphino)-ferrocene]dichloropalladium(II)dichloromethane complex (0.188 g, 0.103 mmol) in toluene (34.5 mL) and ethanol (11.3 mL) was placed under argon. To this solution sodium bicarbonate, 2M (34.5 mL, 69.0 mmol) was added and the mixture was heated at 80 °C for 30 min. Ethyl acetate (44 mL) and (11 mL) water were added to the reaction mixture. The organic extract was concentrated by rotatory evaporation. The crude product was chromatographed on silica gel eluting with 0-40% ethyl acetate in hexane to afford 4.58 g of an off-white solid. mp: 58.0-59.5 °C; 1H NMR (600 MHz, CDC13) δ [ppm]: 7.43-7.40 (m, 3H), 7.35 (m, 1H), 7.31-7.29 (m, 2H), 1H), 7.26 (t, J=7.6 Hz, 1H), 7.20 (dd, J1=7.6 Hz, J2=1.3 Hz, 1H), 4.78 (s, 2H), 2.25 (s, 3H); 13C NMR (151MHz, DMSO-d6) δ [ppm]: 143.0, 142.2, 140.0, 133.8, 129.7, 129.5, 128.2, 127.0, 126.9, 125.7, 64.2, 16.0; IR ν (ATR cm-1): 3365, 3054, 1601, 1469, 1047, 757.

### Example 17: Preparation of 2-chloro-6-methoxy-3-pyridinecarboxaldehyde (6)

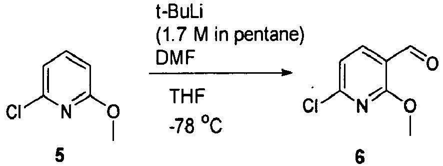

Compound 5 was added (4.14 mL, 34.8 mmol) over 5 min to the solution of tert-butyllithium (1.7M in pentane, 22.5 mL, 38.3 mmol) in 69 mL of THF at -78 °C. The reaction mixture was stirred at -78 °C for 1 h, then dimethylformamide (3.5 mL, 45.0 mmol) was added and the mixture was stirred at -78 °C for 1.5 h. After the addition of glacial acetic acid (4.0 mL, 69.0 mmol), the reaction mixture was allowed to warm to room temperature over a 30-min period and diethyl ether (200 mL) was added. The organic phase was washed with saturated aqueous sodium bicarbonate (50 mL) and brine (50 mL), and was dried over Na2SO4. Concentration under reduced pressure afforded the product which was recrystallized from hexane to yield 4.6 g (76%) of compound 6 as light yellow solid. mp: 67.0-68.0 °C; 1 H NMR (300 MHz, CDC13) δ[ppm]: 10.29 (d, J=0.8 Hz, 1H), 8.05 (d, J=7.9 Hz, 1H), 7.02 (dd, J1=7.9 Hz, J2=0.8 Hz, 1H), 4.08 (s, 3H); 13C NMR (75 MHz, CDC13) δ [ppm]:188.0, 164.2, 154.6, 140.0, 117.6, 117.5, 54.8; IR ν (ATR cm-1): 3103, 2869, 1683, 1567, 1468, 1378, 1273, 1005.

### Example 18: Preparation of 2-methoxy-6-[(2-methyl-3-phenylphenyl)methoxy]pyridine-3-carbaldehyde (7)

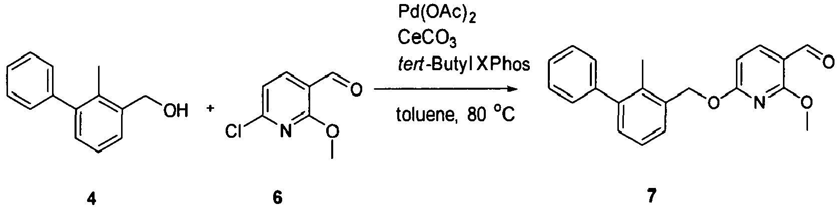

Palladium (II) acetate (0.08 g, 0.36 mmol), caesium carbonate (2.23 g, 6.83 mmol), 2-di-tert-butylphosphino-2',4',6'-triisopropylbiphenyl (t-Butyl XPhos) (0.290 g, 0.68 mmol), compound 6 (0.59 g, 3.41 mmol), and 4 (0.88 g, 4.44 mmol) and toluene (30 mL) were combined and purged by a stream of argon for 3 minutes. The reaction was sealed and heated at 80 °C for 4h. The mixture was filtered through a pad of Celite. The filtrate was concentrated under reduced pressure. The product was purified by flash chromatography on silica gel using 0-60% ethyl acetate in hexane. The product was recrystallized from diethyl ether (488 mg, 35%). mp: 132.0-133.0 °C; 1H NMR (600MHz, CDC13) δ[ppm]: 10.22 (d, J=0.8 Hz, 1H), 8.06 (d, J=8.4 Hz, 1H), 7.44-7.41 (m, 3H), 7.37-7.34 (m, 1H), 7.32-7.30 (m, 2H), 7.28 (d, J=7.5 Hz, 1H), 7.26 (m, 1H), 6.45 (dd, J1=8.4 Hz, J2=0.8 Hz, 1H), 5.52 (s, 2H), 4.08 (3H), 2.28 (s, 3H); 13C NMR (151 MHz, DMSO-d6) δ [ppm]: 187.8, 166.6, 165.2, 143.2, 142.0, 140.5, 135.0, 134.8, 130.5, 129.5, 128.7, 128.3, 127.1, 125.7, 112.5, 103.9, 67.8, 54.1, 16.5; IR ν (ATR cm-1): 3063, 2961, 2870, 1671, 1591, 1460, 1330, 1277; HRMS (ESI-TOF) Calcd for C21H19NO3Na [M+Na]+ : 356.1263; found [M+Na]+: 356.1256.

### Example 19: Preparation of N-{2-[({2-methoxy-6-[(2-methyl-3-phenylphenyl)methoxy]pyridin-3-yl}methyl)amino]ethyl}acetamide hydrochloride BMS-202 (9)

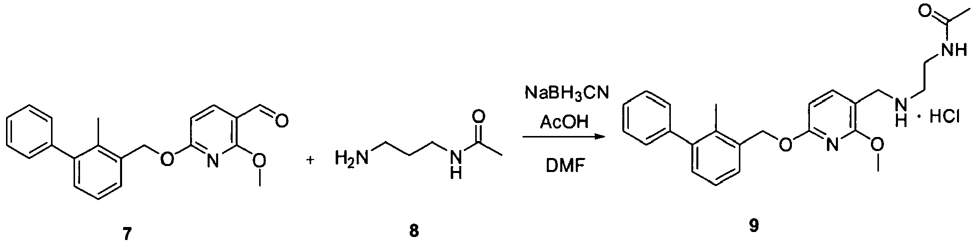

Combined sodium cyanoborohydride (200 mg, 3.18 mmol), N-(2-aminoethyl)acetamide 8 (250 mg, 2.45 mmol), and compound 9 (200 mg, 0.60 mmol) in DMF (20 mL) and acetic acid (5 drops) were stirred at room temperature for 16 h. The mixture was concentrated under reduced pressure. The product was purified by flash chromatography on silica gel (0-60% methanol in ethyl acetate) as yellow oil. The product 9 was converted into the corresponding hydrochloride salt and recrystallized from acetone (76 mg, yield: 27%). mp: 139.5-140.5 °C; 1H NMR (600MHz, DMSO-d6) δ [ppm]: 8.97 (br. s., 2H), 8.20 (t, J=5.6 Hz, 1H), 7.82 (d, J= 8.0 Hz, 1H), 7.47-7.44 (m, 3H), 7.39-7.36 (m, 1H), 7.30 (m, 2H), 7.21 (t, J= 7.6, 1H), 7.19 (dd, J1= 7.7 Hz, J2=1.2 Hz, 1H), 6.53 (d, J=8.0 Hz, 1H), 5.45 (s, 2H), 4.04 (s, 2H), 3.95 (s, 3H), 3.36 (q, J=6.3 Hz, 2H), 2.95 (t, J=6.3 Hz, 2H), 2.22 (s, 3H), 1.83 (s, 3H); 13C NMR (151MHz, DMSO-d6) δ [ppm]: 170.2, 162.5, 160.5, 144.0, 142.2, 141.4, 135.6, 133.9, 129.7, 129.2, 128.3, 128.3, 127.0, 125.5, 105.3, 101.5, 66.5, 53.7, 46.1, 44.1, 35.2, 22.6, 15.9; IR ν (ATR cm-1) 3253, 3062, 2934, 2702, 1651, 1605, 1587,1463, 1309, 1003; HRMS (ESI-TOF) Calcd for C25H29N3O3 [M+H]+: 420.2287; found [M+H]+: 420.2299.

### Example 20: Preparation of 3-bromo-4-[(2-methyl-3-phenylphenyl)methoxy]benzaldehyde (11)

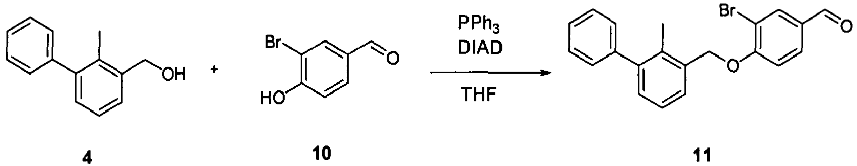

To the ice-cooled solution of 3-bromo-4-hydroxybenzaldehyde 10 (0.71 g, 3.51 mmol), triphenylphosphine (1.02 g, 3.89 mmol) and compound 4 (0.70 g, 0.52 mmol) in dry THF (21 mL), diisopropyl azodicarboxylate (DIAD) (0.735 mL, 3.89 mmol) in THF (21 mL) was added dropwise. The resulting yellow solution was allowed to warm to room temperature and was stirred for additional 20 h. The mixture was concentrated under reduced pressure. The residue was purified by flash chromatography on silica gel using 0-60% ethyl acetate in hexane. Recrystallization from MeOH yielded colourless solid (0.47 g, yield: 35%) .mp: 108.0-110.0 °C; 1H NMR (600MHz, DMSO-d6) δ[ ppm]: 9.87 (s, 1H), 8.14 (d, J=2.0 Hz, 1H), 7.97 (dd, J1= 6.5 Hz, J2=2.0 Hz, 1H), 7.54 (m, 2H), 7.46 (m, 2H), 7.39 (m, 1H), 7.33-7,30 (m, 3H), 7.22 (dd, J1= 6.5 Hz, J2= 1 Hz, 1H), 5.39 (s, 2H), 2.23 (s, 3H); 13C NMR (151MHz, DMSO-d6) δ [ppm]: 190.6, 159.2, 142.2, 141.2, 134.6, 134.0, 133.9, 131.2, 130.8, 129.8, 129.2, 128.3, 127.5, 127.0, 125.6, 114.1, 111.9, 69.8, 15.9; IR ν (ATR cm-1) 3063, 2852, 1689, 1594, 1278, 1254, 1189, 1048; HRMS (ESI-TOF) Calcd C21H17BrO2 [M+Na]+: 403.0310; found [M+Na]+: 403.0302.

### Example 21: Preparation of 1-({3-bromo-4-[(2-methyl-3-phenylphenyl)methoxy]phenyl}methyl)piperidine-2-carboxylic acid BMS-8 (13)

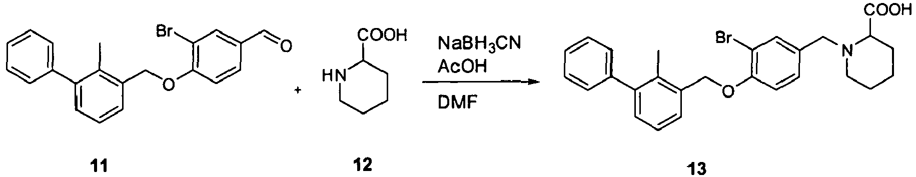

A solution of 3-bromo-4-[(2-methyl-3-phenylphenyl)methoxy]-benzaldehyde 11 (150 mg, 0.39 mmol), piperidine-2-carboxylic acid (148 mg, 1.17 mmol), sodium cyanoborohydride (74 mg, 1.17 mmol) and acetic acid (2 drops) in DMF (4 mL) was stirred at 80 °C for 3 h. The mixture was concentrated under reduced pressure. The residue was purified by silica gel flash chromatography (0-60% methanol in ethyl acetate). The product was recrystallized from ethyl acetate (50 mg, yield: 26%). mp: 119.5-121.0 °C; 1H NMR (300 MHz, DMSO-d6) δ[ppm]: 17.45 (br.s, 1H), 7.73 (d, J=2.0 Hz, 1H), 7.55 (dd, J1=7.5 Hz, J2=1.2 Hz, 1H), 7.49-7.35 (m, 8H), 7.22 (dd, J1=7.5 Hz, J2=1.2 Hz, 1H), 5.29 (s, 2H), 4.62 (s, 2H), 3.78 (m, 1H), 3.21 (m, 2H), 2.23 (s, 3H), 2.03-1.93 (m, 2H), 1.86-1.56 (m, 3H), 1.50-1.34 (m, 1H); 13C NMR (75MHz, DMSO-d6) δ [ppm]: 170.2, 155.7, 137.1, 134.9, 133.8, 133.7, 129.7, 129.2, 128.3, 127.6, 127.0, 125.6, 122.0, 113.7, 110.8, 69.9, 69.4, 68.6, 59.9, 25.0, 20.1, 19.6, 15.8; IR ν (ATR cm-1) 3329, 2946, 2520, 1728,1605, 1500, 1452, 1290, 1265, 1056; HRMS (ESI-TOF) Calcd C25H29N3O3 [M+Na]+: 516.1150; found [M+Na]+: 516.1137.

### Example 21: Preparation of 2,6-dimethoxy-4-[(2-methyl-3-phenylphenyl)methoxy]benzaldehyde (15)

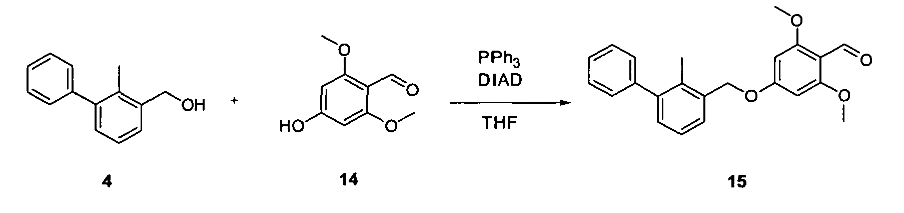

To the ice-cooled solution of 2,6-dimethoxy-4-[(2-methyl-3-phenylphenyl)methoxy]benzaldehyde 14 (0.92 g, 5.04 mmol), triphenylphosphine (1.45 g, 5.55 mmol) and compound 4 (1.0 g, 5.04 mmol) in dry THF (21 mL), diisopropyl azodicarboxylate (DIAD) (1.08 mL, 5.55 mmol) in THF (21 mL) was added dropwise. The resulting yellow solution was allowed to warm to room temperature and stirred for additional 20 h. The mixture was concentrated under reduced pressure. The residue was purified by flash chromatography on silica gel using 0-60% ethyl acetate in hexane as colourless solid (1.02 g, yield: 56%). mp: 161.5-162.0 °C; 1H NMR (600 MHz, DMSO-d6) δ[ppm]: 10.37 (s, 1H), 7.44-7.40 (m, 3H), 7.37 (m, 1H), 7.32 -7.28 (m, 4H), 6.20 (s, 2H), 5.16 (s, 2H), 3.89 (s, 6H), 2.27 (s, 3H); 13C NMR (151 MHz, DMSO-d6) δ [ppm]: 187.9, 165.6, 164.3, 143.4, 141.9, 134.7, 134.3, 130.8, 129.5, 128.5, 128.3, 127.1, 125.9, 109.1, 91.1, 69.7, 56.2, 16.4; IR ν (ATR cm-1) 3013, 2936, 1668, 1607, 1607, 1582, 1465, 1166; HRMS (ESI-TOF) Calcd C23H22O4 [M+Na]+ : 385.1416; found [M+Na]+ : 385.1420

### Example 22: Preparation of N-{2-[({2,6-dimethoxy-4-[(2-methyl-3-phenylphenyl)methoxy]phenyl}methyl)amino]ethyl}acetamide BMS-37 (16)

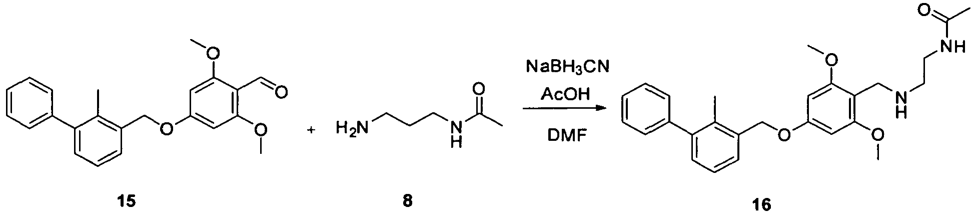

A solution of 2,6-dimethoxy-4-[(2-methyl-3-phenylphenyl)-methoxy]benzaldehyde 15 (90 mg, 0.25 mmol), N-(2-aminoethyl)-acetamide 8 (104 mg, 1.02 mmol), sodium cyanoborohydride (83.5 mg, 1.3 mmol) and acetic acid (2 drops) in DMF (5 mL) was stirred at 80 °C for 3 h. The mixture was concentrated under reduced pressure. The residue was purified by silica gel flash chromatography (0-60% methanol in CHCl3) as colourless solid. (41 mg, yield: 36%). mp: 112.0-113.5 °C; 1H NMR (600 MHz, DMSO-d6) δ[ppm]: 7.75 (t, J=5.4 Hz, 1H), 7.47 (dd, J1=7.6 Hz, J2=1.05 Hz, 1H), 7.45 (m, 2H), 7.38 (m, 1H), 7.32 (m, 2H), 7.29 (t, J=7.6 Hz, 1H), 7.21 (dd, J1=7.6 Hz, J2=1.20 Hz, 1H), 6.36 (s, 2H), 5.15 (s, 2H), 3.76 (s, 6H), 3.59 (s, 2H), 3.08 (q, J=6.3 Hz, 2H), 2.43 (t, J=6.4 Hz, 2H), 2.22 (s, 3H), 1.77 (s, 3H); 13C NMR (151 MHz, DMSO-d6) δ [ppm]:169.0, 159.2, 158.9, 142.2, 141.4, 135.6, 134.0, 129.7, 129.2, 128.2, 127.0, 125.5, 108.7, 91.5, 68.6, 55.7, 47.9, 40.5, 40.1, 38.8, 22.6, 15.9; IR ν (ATR cm-1) 3317, 2934, 2836, 1647, 16155, 1597, 1498, 1200, 1147, 1033; HRMS (ESI-TOF) Calcd C27H32N2O4 [M+H]+: 449.2440; found [M+H]+: 449.2440

### Example 23: Preparation of (2R)-2-[({2,6-dimethoxy-4-[(2-methyl-3-phenylphenyl)methoxy]-phenyl}methyl)amino]-3-methylbutan-1-ol BMS-242 (17)

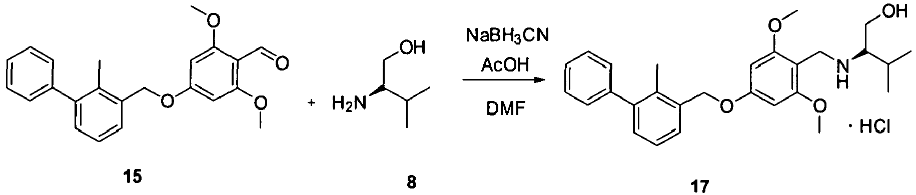

A solution of 2,6-dimethoxy-4-[(2-methyl-3-phenylphenyl)-methoxy]-benzaldehyde 15 (181 mg, 0.5 mmol), L-valinol 8 (210 mg, 2.03 mmol), sodium cyanoborohydride (167 mg, 2.6 mmol) and acetic acid (2 drops) in DMF (4 mL) was stirred at 80 °C for 3 h and in room temperature overnight. The mixture was concentrated under reduced pressure. The residue was purified by silica gel flash chromatography (0-60% methanol in ethyl acetate). The product 17 was converted into the corresponding hydrochloride salt and recrystallized from isopropanol and diisopropyl ether (55 mg, yield: 48 %). mp: 74.0-76.0 °C; 1H NMR (600 MHz, DMSO-d6) δ[ppm]: 8.08 (d br., J=54.5 Hz, 2H), 7.5 (dd, J1=6.6 Hz, J2=1.0 Hz, 1H), 7.46 (m, 2H), 7.38 (m, 1H), 7.31 (m, 3H), 7.21 (dd, J1=6.6 Hz, J2=1.20 Hz, 1H), 6.46 (s, 2H), 5.34 (t, J=4.9 Hz, 1H), 5.21 (s, 2H), 4.12 (t, J= 5.6 Hz, 2H), 3.83 (s, 6H), 3.70 (m, 1H), 2.80 (m, 1H), 2.22 (s, 3H), 2.08 (m, 1H), 1.23 (s, 1H), 0.96 (d, J= 6.9 Hz, 3H), 0.92 (d, J= 6.9 Hz, 3H); (Peaks observed at 1.04 and 3.61 ppm corresponds to residual amount of diisopropyl ether) 13C NMR (151 MHz, DMSO-d6) δ [ppm]: 161.9, 159.9, 142.7, 141.8, 135.8, 134.6, 130.3, 129.6, 128.8, 128.7, 127.5, 126.0, 100.2, 92.0, 69.3, 67.8, 64.5, 57.7, 56.5, 38.7, 26.8, 23.3, 19.6, 18.1, 16.4; IR ν (ATR cm-1) 3289 (br), 2965, 1611, 1595, 1463, 1150; HRMS (ESI-TOF) Calcd C28H35NO4 [M+H]+: 450.2644; found [M+H]+: 450.2640

### Further examples:

| **example** | **smiles** | **MH⁺** |
|---|---|---|
| 23 | CC1=C(C=CC=C1C2=CC=CC=C2)OCC3=CC(C#N)=C(N)S3 | 321.1 |
| 24 | CC1=C(C=CC=C1C2=CC=CC=C2)OCC3=C(CC4=CC=CC=C4) SC(N)=C3C#N | 411.2 |
| 25 | CC1=C(C=CC=C1C2=CC=CC=C2)OCC3=CC(CN4CCCC4)=C( N)S3 | 379.2 |
| 26 | CC1=C(C=CC=C1C2=CC=CC=C2)OCC3=CC(CN)=C(N)S3 | 325.1 |
| 27 | CC1=C(C=CC=C1C2=CC=CC=C2)OCC3=CC(CN4CCOCC4)=C (N)S3 | 395.2 |
| 28 | CC1=C(C=CC=C1C2=CC=CC=C2)OCC3=CC(C#N)=C(N4CCN CC4)S3 | 390.2 |
| 29 | CC1=C(C=CC=C1C2=CC=CC=C2)OCC3=CC(C#N)=C(N4CCO CC4)S3 | 391.1 |
| 30 | CC1=C(C=CC=C1C2=CC=C3C(OCCO3)=C2)OCC4=CC5=C(N CNC5)S4 | 395.5 |

| **example** | **smiles** | **MH⁺** |
|---|---|---|
| 31 | CC1=C(CN)N=C(S1)COC2=NC=CC(C3=CC=CC=C3)=C2C#N | 337.1 |
| 32 | CC1=C(CNCCNC(C)=O)N=C(S1)COC2=NC=CC(C3=CC=CC= C3)=C2C#N | 422.2 |
| 33 | FC1=CC(C2=C(CN3CCN(C(C)=O)CC3)N=C(S2)COC4=NC= CC(C5=CC=CC=C5)=C4C#N)=CC(F)=C1F | 564.2 |
| 34 | CC1=C(CNCCNC(C)=O)N=C(S1)COC2=NC=CC(C3=CC(OCC O4)=C4C=C3)=C2C#N | 480.2 |
| 35 | ClC(C=C1)=CC=C1C2=C(CN3CCN(CCO)CC3)N=C(S2)COC 4=NC=CC(C5=CC=CC=C5)=C4C#N | 546.2 |
| 36 | CC(NCCNCC1=C(CC2=CC=CC=C2)SC(COC3=NC=CC(C4=CC (OCCO5)=C5C=C4)=C3C#N)=N1)=O | 556.2 |
| 37 | NCC1=CSC(COC2=NC=CC(C3=CC(OCCO4)=C4C=C3)=C2C# N)=N1 | 381.1 |
| 38 | NCC1=CSC(COC2=NC=CC(C3=CC(OCO4)=C4C=C3)=C2C#N )=N1 | 367.1 |
| 39 | N[C@H](C)C1=CSC(COC2=NC=CC(C3=CC=CC=C3)=C2C#N )=N1 | 337.1 |
| 40 | N[C@@H](C)C1=CSC(COC2=NC=CC(C3=CC=CC=C3)=C2C# N)=N1 | 337.1 |

| **example** | **smiles** | **MH⁺** |
|---|---|---|
| 41 | NNCC1=CSC(COC(N=CC=C2C3=CC=CC=C3)=C2C#N)=N1 | 338.1 |
| 42 | N#CC1=C(N=CC=C1C2=CC=C3C(OCCO3)=C2)OCC4=NC(CN N5CCOCC5)=CS4 | 466.2 |
| 43 | OCC(C(N)=O)NCC1=CSC(COC(N=CC=C2C3=CC=C4C(OCCO 4)=C3)=C2C#N)=N1 | 468.1 |
| 44 | N#CC1=C(N=CC=C1C2=CC=C3C(OCCO3)=C2)OCC4=NC(CN 5CCOCC5)=CS4 | 451.1 |
| 44 | O=S(CC1)CCN1CC2=CSC(COC(N=CC=C3C4=CC=C5C(OCCO 5)=C4)=C3C#N)=N2 | 483.1 |
| 46 | O=S(CCN1CC2=CSC(COC(N=CC=C3C4=CC=C5C(OCCO5)=C 4)=C3C#N)=N2)(CC1)=O | 499.1 |
| 47 | N#CC1=C(N=CC=C1C2=CC=C3C(OCCO3)=C2)OCC4=NC(C5 =CC=CC=C5)=C(CN6CCOCC6)S4 | 527.1 |
| 48 | O=S(N(CC1)CCCN1CC2=CN=C(S2)COC(N=CC=C3C4=CC=C 5C(OCCO5)=C4)=C3C#N)(C)=O | 542.2 |
| 49 | CC(N(CC1)CCCN1CC2=C(C)N=C(S2)COC(N=CC=C3C4=CC =C5C(OCCO5)=C4)=C3C#N)=O | 520.2 |
| 50 | ClC1=CC=C(C2=CC=NC(OCC3=NC(CN4CCOCC4)=C(C5=CC =CC=C5)S3)=C2C#N)C=C1 | 503.1 |

| **example** | **smiles** | **MH⁺** |
|---|---|---|
| 51 | O=C(NCCN1CCOCC1)C2=C(C3=CC=CC=C3)N=C(O2)COC(N =CC=C4C5=CC=CC=C5)=C4C#N | 510.2 |
| 52 | N#CC1=C(N=CC=C1C2=CC=CC=C2)OCC3=NC(C4=CC=CC=C | 496.2 |
| | 4)=C(CNCCN5CCOCC5)O3 | |
| 53 | FC1=CC=CC=C1C2=C(CNCCN3CCOCC3)OC(COC(N=CC=C4C 5=CC=CC=C5)=C4C#N)=N2 | 514.2 |
| 54 | FC1=CC(C2=C(CNCCN3CCOCC3)OC(COC(N=CC=C4C5=CC= CC=C5)=C4C#N)=N2)=CC=C1 | 514.2 |
| 55 | FC(C=C1)=CC=C1C2=C(CNCCN3CCOCC3)OC(COC(N=CC=C 4C5=CC=CC=C5)=C4C#N)=N2 | 514.2 |
| 56 | CC1=C(CNCCCNC(C)=O)OC(COC(N=CC=C2C3=CC=CC=C3) =C2C#N)=N1 | 420.2 |
| 57 | CC(NCCNCC1=C(C2=CC=CC=C2)N=C(O1)COC(N=CC=C3C4 =CC=CC=C4)=C3C#N)=O | 468.2 |
| 58 | N#CC1=C(N=CC=C1C2=CC=CC=C2)OCC3=NC(C4=CC=CC=C 4)=C(CNCCN5CCOCC5)N3CCC6=CC=CC=C6 | 599.3 |
| 59 | CC1=C(CNCCNC)N(CCO)C(COC(N=CC=C2C3=CC=C4C(OCC O4)=C3)=C2C#N)=N1 | 479.2 |
| 60 | N#CC1=C(N=CC=C1C2=CC=CC=C2)OCC3=NC(C4=CC=C(C5 =CC=CC=C5)C=C4)=C(CNCCN6CCOCC6)O3 | 572.3 |
| 61 | N[C@H]([C@H](O)C1)C2=C1C=CC(C3=C(C#N)C(C4=CC( OCO5)=C5C=C4)=CS3)=C2 | 377.1 |
| 62 | N[C@H]([C@@H](CC1)O)C2=C1C=CC(C3=C(C#N)C(C4=C C(OCCO5)=C5C=C4)=CS3)=C2 | 407.1 |
| 63 | COC(C=C1)=C(OC)C=C1C2=CSC(C3=CC([C@H](N)[C@@H ](CC4)O)=C4C=C3)=C2C#N | 377.1 |
| 64 | COC(C=C1)=CC=C1C2=CSC(C3=CC([C@H](N)[C@@H](C4 )N)=C4C=C3)=C2C#N | 362.1 |

### Screening:

### Expression and purification of recombinant PD-L1, PD-L2 and PD-1

The gene encoding human PD-L1 (amino acids 18-134) was cloned into the pET-21b, the gene encoding human PD-L2 (20-220) was cloned into pET28a and that of human PD-1 (33-150, Cys93 exchanged to serine) into pET-24d. Proteins were expressed in the E. coli BL21 (DE3). Cells were cultured in LB at 37°C. The protein production was induced with 1 mM IPTG at OD600 of 1.0 and the cells were cultured for additional 5h. For hPD-1, after induction the temperature was lowered to 30oC. Proteins were expressed as inclusion bodies which were collected by centrifugation, washed twice with 50 mM Tris-HCl pH 8.0 containing 200 mM NaCl, 0.5% Triton X-100, 10 mM EDTA and 10 mM 2-mercaptoethanol and once more with the same buffer with no detergent. The inclusion bodies were stirred overnight in 50 mM Tris pH 8.0 containing 6M GuHCl, 200 mM NaCl and 10 mM 2-mercaptoethanol. Solubilized fraction was clarified by high speed centrifugation. hPD-L1 and hPD-L2 were refolded by dropwise dilution into 0.1 M Tris pH 8.0 containing 1 M L-Arg hydrochloride, 0.25 mM oxidized glutathione and 0.25 mM reduced glutathione for hPD-L1 and 0.1 M Tris pH 8.5 containing 1 M NDSB201, 0.2 M NaCl, 5 mM cysteamine and 0.5 mM cystamine for hPD-L2. hPD-1 was refolded in similar manner in 0.1 M Tris pH 8.0 containing 0.4 M L-Arg hydrochloride, 2 mM EDTA, 5 mM cystamine and 0.5 mM cysteamine. After refolding, the proteins were dialyzed 3 times against 10 mM Tris pH 8.0 containing 20 mM NaCl, and purified by size exclusion chromatography on Superdex 75 (GE Healthcare) in 10 mM Tris pH 8.0 containing 20 mM NaCl. The purity and protein folding were evaluated by SDS-PAGE and NMR, respectively.

### Analytical size-exclusion chromatography

The oligomeric state of tested proteins was analyzed by size exclusion chromatography. Superdex 75 10/30 HR (GE Healthcare) was equilibrated with PBS pH 7.4 and calibrated using globular proteins of known molecular weight. Approximate molecular weight of apo-hPD-L1 and hPD-L1-small molecule complex (3:1 compound : protein molar ratio) were estimated using the calibration curve.

### Differential scanning fluorimetry (DSF)

DSF analysis was performed according to Niesen and colleagues (24). In brief hPD-L1 and hPD-L2 (both 12.5 µM) were incubated alone, with compound BMS-202 or compound 8 (both at 37.5 µM) in the presence of SYPRO Orange Dye (Life Technologies, final concentration 20x). Constant temperature gradient of 0.2°C/min was applied and changes in fluorescence were monitored using real time thermocycler (BioRad). Melting temperature (Tm) was estimated from first derivative of fluorescence intensity as a function of temperature.

### NMR methods

Uniform 15N labeling was obtained by expressing the protein in the M9 minimal medium containing 15NH4C1 as the sole nitrogen source. Unlabeled proteins were prepared as for crystallization. For NMR measurements the buffer was exchanged by gel filtration to PBS pH 7.4 (hPD-L1) or 25 mM sodium phosphate containing 100 mM NaCl pH 6.4 (hPD-1). 10% (v/v) of D2O was added to the samples to provide lock signal. All spectra were recorded at 300K using a Bruker Avance 600 MHz spectrometer.

Binding of the compounds was analyzed by titrating the 15N-labeled PD-L1 (0.3 mM) and recording the 1H-15N HMQC spectra prior and after addition of the compound (Supplementary Figs. S1, S2 and S3).

The ability of tested compounds to dissociate hPD-L1 / hPD-1 was evaluated using AIDA (27). 15N-labeled hPD-1 (0.2 mM) was slightly overtitrated with unlabeled hPD-L1. Compound was aliquoted into the resulting mixture. During the experiment the 1H-15N signals were monitored by HMQC experiment.

Changes in the oligomeric state of hPD-L1 in the presence of tested compounds were monitored by titration of unlabeled hPD-L1 (0.3 mM) while recording 1H spectra prior and after addition of the compound. The approximate molecular weights of protein populations present in the sample were determined by analyzing the linewidth (relaxation time) of well separated NMR signals.

The compounds showed activity (IC₅₀) in the range of from 0.001 to 1000 µM.

## Claims

1. A compound of formula (I): 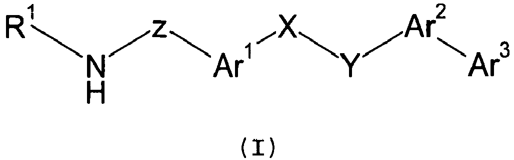 wherein
R¹ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
Ar¹ is an aryl, hereroaryl, aralkyl or heteroaralkyl group, all of which may optionally be substituted;
Ar² is a phenylene group or a heteroarylene group having 5 or 6 ring atoms and 1, 2, 3 or 4 heteroatoms selected from O, S and N, which group Ar² is unsubstituted or substituted by one group R²;
Ar³ is a phenylene group or a heteroarylene group having 5 or 6 ring atoms and 1, 2, 3 or 4 heteroatoms selected from O, S and N which group Ar³ is unsubstituted or substituted by one, two or three group(s) R³;
X is a bond or O, S, CHOMe, NOMe, CFH, CF₂, NH or CH₂;
Y is a bond or O, S, CHOMe, NOMe, CFH, CF₂, NH or CH₂;
z is a bond or a C₁-C₆ alkylene, a C₂-C₆ alkenylene, a C₂-C₆ alkynylene or a heteroalkylene group containing from 2 to 8 atoms selected from C, N, O and S;
R² is methyl, CN or halogen;
the group(s) R³ is/are independently halogen, CN, hydroxy or a C₁-C₆ alkyl, a C₂-C₆ alkenyl, a C₂-C₆ alkynyl or a heteroalkyl group containing from 2 to 8 atoms selected from C, N, O and S or a C₃-C₇ cycloalkyl group or a heterocycloalkyl group containing 3 to 7 ring atoms selected from O, S, N and C; or
two groups R³ together are part of a C₃-C₇ cycloalkyl group, a heterocycloalkyl group containing 3 to 7 ring atoms selected from O, S, N and C, a phenyl group or a heteroraryl group having 5 or 6 ring atoms and 1, 2, 3 or 4 heteroatoms selected from O, S and N;
or a pharmaceutically acceptable salt, ester, solvate or hydrate or a pharmaceutically acceptable formulation thereof.

2. A compound according to claim 1, wherein X is CH₂ and Y is O or X is O and Y is CH₂.

3. A compound according to claim 1 or 2, wherein z is CH₂.

4. A compound according to any one of the preceding claims, wherein R¹ is hydrogen, methyl or a group of formula CH₂CH₂OH, CH₂CH₂NH₂ or CH₂CH₂NHCOCH₃.

5. A compound according to any one of the preceding claims, wherein Ar¹ is selected from the following groups: 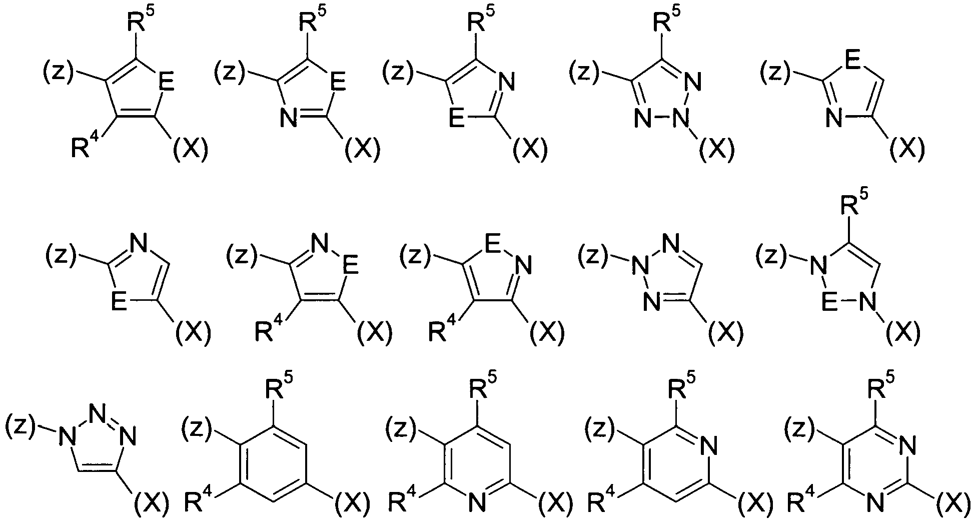 wherein (z) denotes the bond to group z; (X) denotes the bond to group X; E is selected from O, S and NR⁶; and R⁴, R⁵ and R⁶ are independently selected from a hydrogen atom, a halogen atom, NO₂, N₃, OH, SH, NH₂, SO₃H or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

6. A compound according to any one of the preceding claims, wherein, Ar² is selected from the following groups: 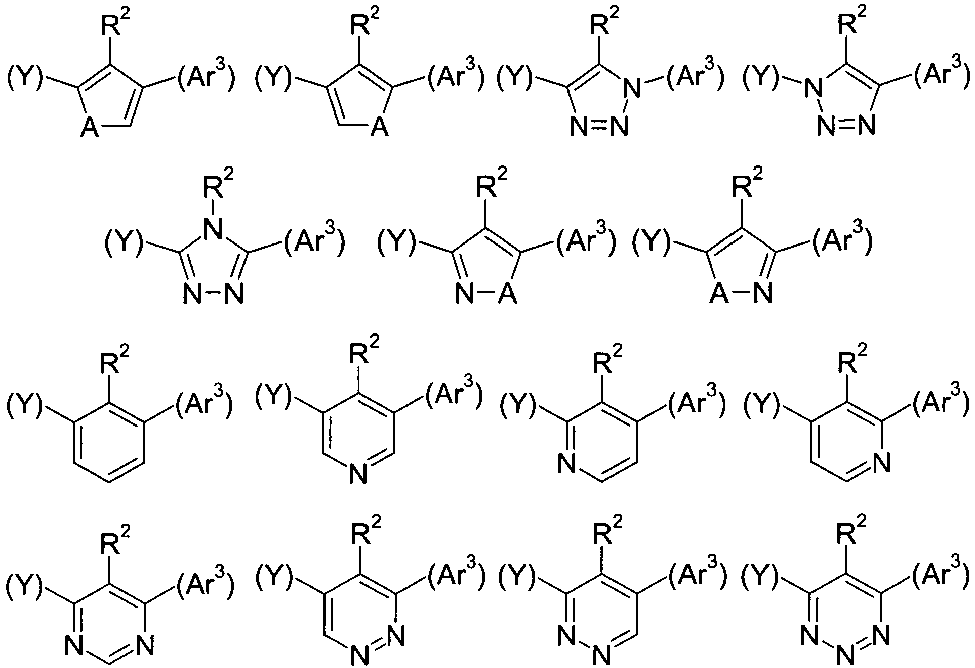 wherein (Y) denotes the bond to group Y; (Ar³) denotes the bond to group Ar³; A is selected from O, S and NH and R² is as defined above.

7. A compound according to any one of the preceding claims, wherein Ar³ is selected from the following groups: 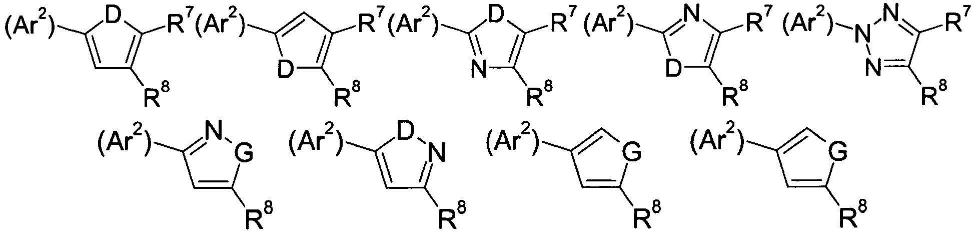 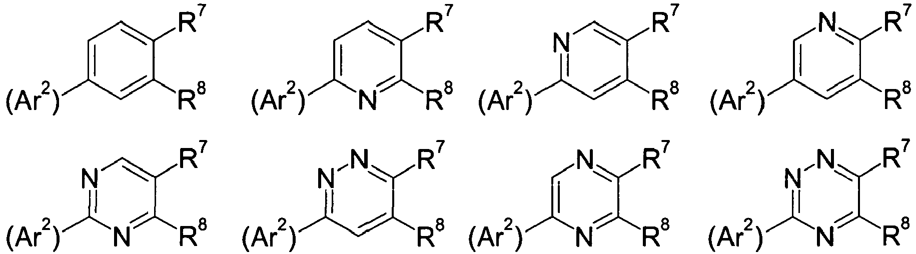 wherein (Ar²) denotes the bond to group Ar²; D is selected from O, S and NR⁹; G is selected from O, S and NR⁷; R⁷, R⁸ and R⁹ are independently hydrogen, halogen, CN, hydroxy or a C₁-C₆ alkyl, a C₂-C₆ alkenyl, a C₂-C₆ alkynyl or a heteroalkyl group containing from 2 to 8 atoms selected from C, N, O and S or a C₃-C₇ cycloalkyl group or a heterocycloalkyl group containing 3 to 7 ring atoms selected from O, S, N and C; or R⁷ and R⁸ together are part of a C₃-C₇ cycloalkyl group, a heterocycloalkyl group containing 3 to 7 ring atoms selected from O, S, N and C, a phenyl group or a heteroraryl group having 5 or 6 ring atoms and 1, 2, 3 or 4 heteroatoms selected from O, S and N.

8. A compound according to any one of the preceding claims, wherein Ar³ is unsubstituted or wherein Ar³ is substituted by a halogen atom.

9. A compound according to any one of the preceding claims, wherein Ar³ is substituted by two groups that together are part of a C₅-C₆ cycloalkyl group or a heterocycloalkyl group containing 5 or 6 ring atoms selected from O, S, N and C.

10. A compound according to any one of the preceding claims, wherein Ar³ is substituted by two groups which together form a group of formula -O-CH₂CH₂-O-, -O-CH₂-O- or -O-CF₂-O-.

11. A compound according to any one of the preceding claims, wherein R² is methyl.

12. A compound of formula (I) according to claim 1: 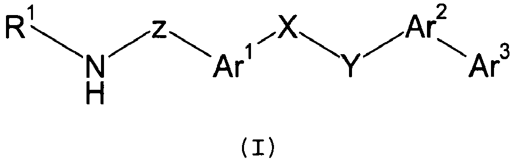 wherein
R¹ is hydrogen, methyl or a group of formula CH₂CH₂OH, CH₂CH₂NH₂ or CH₂CH₂NHCOCH₃;
X is CH₂ and Y is O or X is O and Y is CH₂;
z is CH₂;
Ar¹ is selected from the following groups: 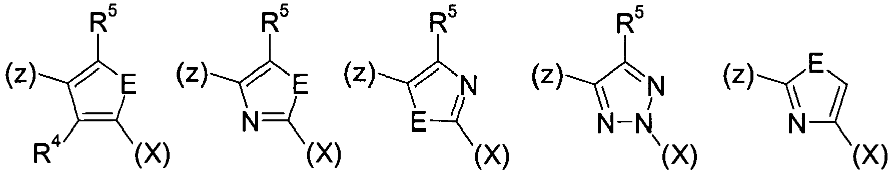 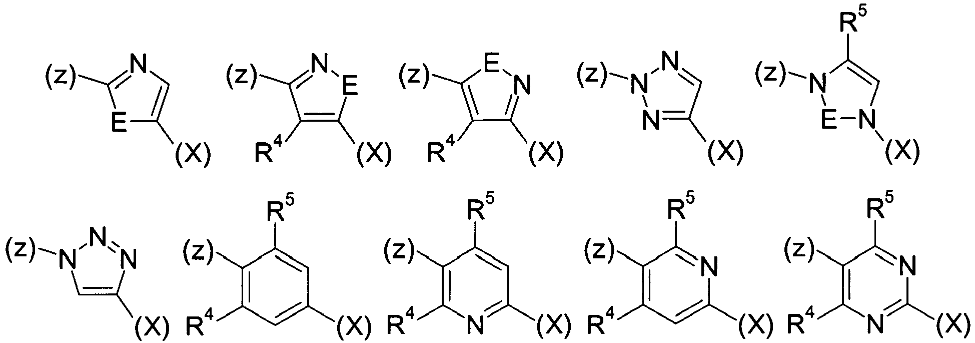 wherein (z) denotes the bond to group z; (X) denotes the bond to group X; E is selected from O, S and NR⁶; and R⁴, R⁵ and R⁶ are independently selected from a hydrogen atom, a halogen atom, NO₂, N₃, OH, SH, NH₂, SO₃H or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
Ar² is selected from the following groups: 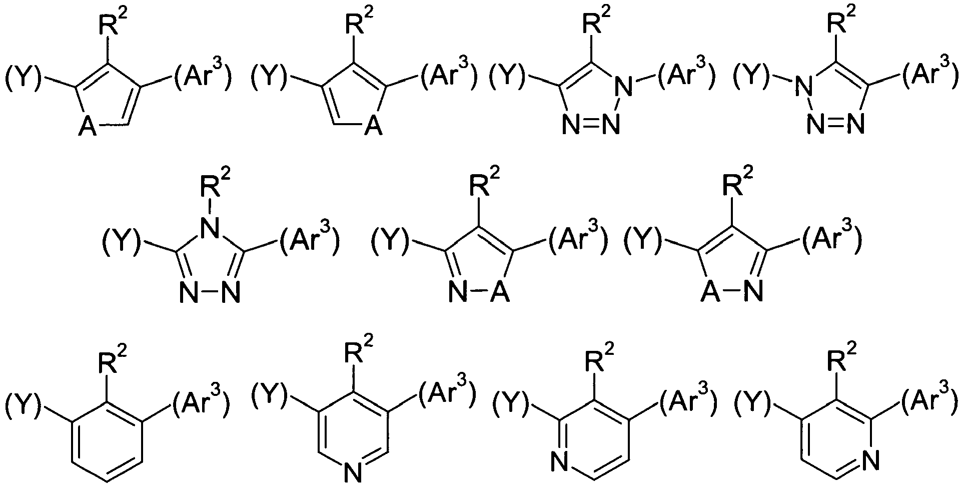 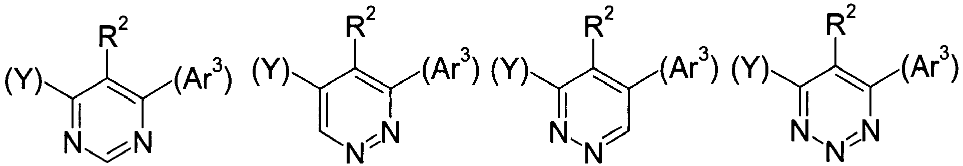 wherein (Y) denotes the bond to group Y; (Ar³) denotes the bond to group Ar³; R² is methyl, CN or halogen; and A is selected from O, S and NH;
Ar³ is selected from the following groups: 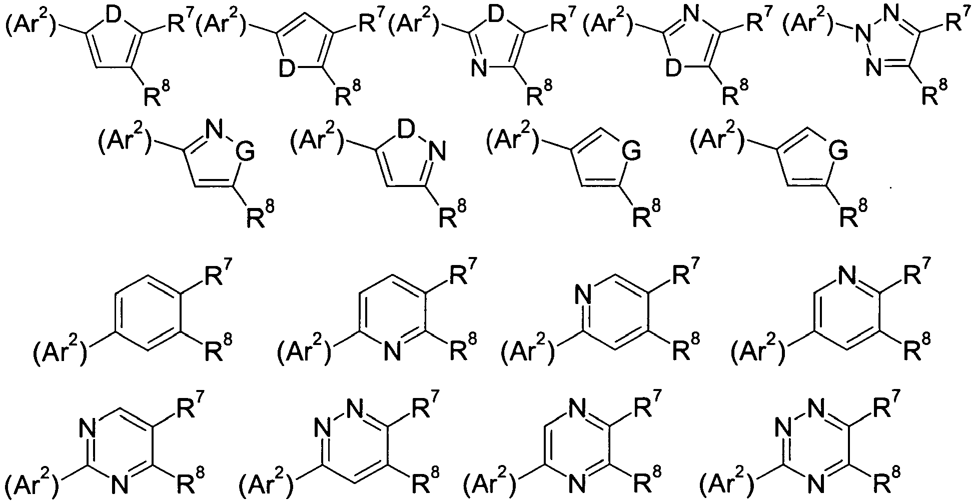 wherein (Ar²) denotes the bond to group Ar²; D is selected from O, S and NR⁹; G is selected from O, S and NR⁷; R⁷, R⁸ and R⁹ are independently hydrogen or halogen, or R⁷ and R⁸ together form a group of formula -O-CH₂CH₂-O-, -O-CH₂-O-, or -O-CF₂-O-;
or a pharmaceutically acceptable salt, ester, solvate or hydrate or a pharmaceutically acceptable formulation thereof.

13. A pharmaceutical composition comprising a compound according to anyone of the preceding claims or a pharmaceutically acceptable ester, prodrug, hydrate, solvate or salt thereof, optionally in combination with a pharmaceutically acceptable carrier.

14. Use of a compound or a pharmaceutical composition according to anyone of the preceding claims for the preparation of a medicament for the treatment of cancer and infectious diseases.

15. A compound or a pharmaceutical composition according to anyone of claims 1 to 14 for use in the treatment of cancer and infectious diseases.
